Europäisches Patentamt

European Patent Office

Office européen des brevets

(19)

(11) **EP 0 700 893 B1**

(12) **EUROPÄISCHE PATENTSCHRIFT**

(45) Veröffentlichungstag und Bekanntmachung des Hinweises auf die Patenterteilung:
**28.01.1998 Patentblatt 1998/05**

(51) Int. Cl.$^6$: **B01J 8/06**, C07C 57/04, C07C 51/25

(21) Anmeldenummer: **95113509.4**

(22) Anmeldetag: **29.08.1995**

(54) **Verfahren zur katalytischen Gasphasenoxidation von Acrolein zu Acrylsäure**

Process for the catalytic vapour phase oxidation of acrolein to acrylic acid

Procédé d'oxydation catalytique en phase gazeuse de l'acroléine en acide acrylique

(84) Benannte Vertragsstaaten:
**BE DE ES FR GB IT NL**

(30) Priorität: **08.09.1994 DE 4431949**

(43) Veröffentlichungstag der Anmeldung:
**13.03.1996 Patentblatt 1996/11**

(73) Patentinhaber:
**BASF AKTIENGESELLSCHAFT**
**67056 Ludwigshafen (DE)**

(72) Erfinder:
• **Ruppel, Wilhelm, Dr.**
**D-67227 Frankenthal (DE)**
• **Wegerle, Ulrike**
**D-67550 Worms (DE)**
• **Tenten, Andreas, Dr.**
**D-67434 Neustadt (DE)**
• **Hammon, Ulrich, Dr.**
**D-68165 Mannheim (DE)**

(56) Entgegenhaltungen:
DE-A- 1 039 040  DE-A- 1 601 162
DE-A- 1 675 501  DE-A- 2 201 528
DE-A- 2 310 517  DE-A- 2 830 765

## Beschreibung

Die vorliegende Erfindung betrifft ein neues Verfahren zur katalytischen Gasphasenoxidation von Acrolein zu Acrylsäure in einem Vielkontaktrohr-Festbettreaktor durch dessen die Kontaktrohre umgebenden Raum lediglich ein Wärmeaustauschmittelkreislauf geleitet wird, bei erhöhter Temperatur an katalytisch aktiven Multimetalloxiden mit einem Umsatz des Acroleins bei einfachem Durchgang von $\geq$ 95 mol.-% und einer Selektivität der Acrylsäurebildung von $\geq$ 90 mol.-%.

Die katalytische Gasphasenoxidation von Acrolein zu Acrylsäure ist allgemein bekannt und insbesondere als zweite Oxidationsstufe bei der Herstellung von Acrylsäure durch zweistufige katalytische Gasphasenoxidation von Propen in zwei hintereinandergeschalteten Reaktionsstufen von Bedeutung (vgl. z.B. DE-A 30 02 829). Acrylsäure ist ein bedeutendes Monomeres, das als solches oder in Form seiner Alkylester zur Erzeugung von z.B. als Klebstoffen geeigneten Polymerisaten Verwendung findet.

Die Gasphasenoxidation von Acrolein zu Acrylsäure verläuft stark exotherm, weshalb es infolge einer Vielfalt von möglichen Parallel- oder Folgereaktionen im Hinblick auf eine möglichst selektive Umsetzung von Acrolein zu Acrylsäure und für eine beherrschbare Durchführung der Gasphasenoxidation überhaupt erforderlich ist, den Verlauf der Reaktionstemperatur in einem gewissen Umfang zu steuern.

Eine ganz allgemein angewandte Möglichkeit der Lenkung der freiwerdenden Reaktionswärme besteht darin, die Reaktionspartner Sauerstoff und Acrolein mit Inertgasen wie $N_2$, Kohlenoxiden wie $CO_2$ und CO, Kohlenwasserstoffen, rückgeführten Reaktionsabgasen und/oder Wasserdampf zu verdünnen, wobei die Verwendung von Verdünnungsgasen mit möglichst hoher molarer Wärmekapazität besonders vorteilhaft ist (vgl. EP-B 253 409).

Eine weitere generell angewandte Methode zur Steuerung der Reaktionstemperatur besteht darin, die katalytische Gasphasenoxidation von Acrolein zu Acrylsäure in einem Vielkontaktrohr-Festbettreaktor durchzuführen. Solche Reaktoren entsprechen in ihrem Typ den Mantel-Rohr-Wärmeaustauschern. D.h. ihre übliche Bauart besteht aus einem, in der Regel zylinderförmigen, Behälter, in welchem eine Vielzahl von Rohren (ein Rohrbündel) entsprechend den Kühlrohren eines Mantel-Rohr-Wärmeaustauschers in üblicherweise vertikaler Anordnung untergebracht ist. Diese Kontaktrohre, von denen ein jedes eine Festbettanordnung des entsprechenden katalytisch aktiven Multimetalloxides enthält, sind mit ihren Enden in Rohrböden abdichtend befestigt und münden in je eine am oberen bzw. unteren Ende mit dem Behälter verbundene Haube. Über diese Hauben wird das die Kontaktrohre durchströmende Reaktionsgasgemisch zubzw. abgeführt, so daß jedes Kontaktrohr einer langgestreckten Reaktionseinheitszone entspricht.

Ferner werden durch den die Kontaktrohre umgebenden Raum Wärmeaustauschmittel geleitet, um die Prozeßwärme zu bewältigen. Nach dem Austritt aus dem Behälter werden die Wärmeaustauschmittel, z.B. in äußeren Wärmetauschern, wieder auf ihre ursprüngliche Temperatur gebracht, bevor sie wieder in den Reaktionsbehälter eintreten (vgl. z.B. DE-A 30 242 468).

Tritt längs der Kontaktrohre auf unterschiedlichen (mehreren) Höhen Wärmeaustauschmittel in den Reaktor ein, so soll hier von einer Anwendung mehrerer Wärmeaustauschmittelkreisläufe gesprochen werden. Erfolgt der Eintritt des Wärmeaustauschmittels lediglich auf einer Höhe, wird hier von einem Wärmeaustauschmittelkreislauf gesprochen, selbst wenn dieser Kreislauf nicht mit einer, sondern aus Gründen der Zweckmäßigkeit mit mehreren Pumpen betrieben wird.

Üblicherweise sind die Kontaktrohre aus ferritischem Stahl gefertigt und weisen in typischer Weise eine Wanddicke von 1 bis 3 mm auf. Ihr Innendurchmesser beträgt in der Regel 20 bis 30 mm. Die Rohrlänge erstreckt sich im Normalfall auf wenige Meter (typisch ist eine Kontaktrohrlänge im Bereich von 2 bis 4 m). Anwendungstechnisch zweckmäßig beläuft sich die im Behälter untergebrachte Anzahl an Kontaktrohren auf wenigstens 5000, vorzugsweise auf wenigstens 10000. Häufig beträgt die Anzahl der im Reaktionsbehälter untergebrachten Kontaktrohre 15000 bis 30000. Rohrbündelreaktoren mit einer oberhalb von 40000 liegenden Anzahl an Kontaktrohren bilden eher die Ausnahme. Innerhalb des Behälters sind die Kontaktrohre im Normalfall homogen verteilt angeordnet, wobei die Verteilung zweckmäßig so gewählt wird, daß der Abstand der zentrischen Innenachsen von zueinander nächstliegenden Kontaktrohren (die sogenannte Kontaktrohrteilung) 35 bis 45 mm beträgt (vgl. z.B. EP-468 290). Als Wärmeaustauschmittel eignen sich insbesondere fluide Temperiermedien. Besonders günstig ist die Verwendung von Schmelzen von Salzen wie Kaliumnitrat, Kaliumnitrit, Natriumnitrit und/oder Natriumnitrat, oder von niedrig schmelzenden Metallen wie Natrium, Quecksilber sowie Legierungen verschiedener Metalle.

Aus der DE-A 26 35 031 ist bekannt, bei Acroleinumsätzen bei einfachem Durchgang von über 95 mol.-% den Verlauf der Reaktionstemperatur der katalytischen Gasphasenoxidation von Acrolein zu Acrylsäure in einem Vielkontaktrohr-Festbettreaktor so zu steuern, daß man die Kontaktrohre mit einer 270°C aufweisenden Salzschmelze umgibt.

Die DE-A 30 42 468 und die DE-A 30 02 829 empfehlen zur Glättung der Temperaturverteilung innerhalb der Katalysatorbetten das Wärmeaustauschmittel und das Reaktionsgasgemisch im Gleichstrom durch den Vielkontaktrohr-Festbettreaktor zu führen. Dabei empfiehlt der Stand der Technik, um möglichst alle Kontaktrohre gleichmäßig am Reaktionsgeschehen zu beteiligen, in einem waagerechten Schnitt des Reaktors (senkrecht zur Reaktorachse) eine möglichst homogene Temperatur des Wärmeaustauschmittels anzustreben (z.B. DE-PS 1 601 162). Des weiteren empfiehlt der Stand der Technik, das Wärmeaustauschmittel möglichst zügig durch den Reaktor zu leiten, um so die freiwerdende Reaktionswärme möglichst effektiv abzuführen. Empfohlen wird, das Wärmeaustauschmittel so im Kreis zu führen, daß der Temperaturunterschied des eingesetzten Wärmeaustauschmittels zwischen Eintritts- und Austrittsstelle aus dem Reaktor möglichst verschwindend ist.

Ein generelles Problem der katalytischen Gasphasenoxidation von Acrolein zu Acrylsäure im Vielkontaktrohr-Festbettreaktor besteht darin, daß die Reaktionstemperatur in Strömungsrichtung längs eines Kontaktrohres ein Maximum, einen sogenannten Heißpunkt, durchläuft. Dies mindert einerseits in diesem Kontaktrohrabschnitt die Lebensdauer des Katalysators und beeinträchtigt andererseits die Selektivität der Acrylsäurebildung.

Verschiedene Gegenmaßnahmen zur Überwindung des genannten Nachteils werden im Stand der Technik bereits empfohlen. Ein Vorschlag besteht in der Verkleinerung des Durchmessers der Kontaktrohre, um so die Wärmeableitung je Volumeneinheit des Katalysators zu erhöhen. Nachteilig an dieser Methode ist jedoch, daß sie die für eine bestimmte Produktionsleistung erforderliche Anzahl katalysatorgefüllter Kontaktrohre in notwendiger Weise erhöht, was sowohl die Fertigungskosten des Reaktors als auch die zum Füllen und Entleeren der Kontaktrohre mit Katalysator erforderliche Zeitdauer steigert.

Nach einem anderen vorgeschlagenen Verfahren wird die Ausbildung der Heißpunkte dadurch zu unterdrücken versucht, daß man die volumenspezifische Aktivität der katalytischen Beschickung längs der Kontaktrohre variiert. Diese Verfahrensweise erfordert jedoch in notwendiger Weise die Anwendung entweder mindestens zweier Katalysatoren unterschiedlicher Aktivität oder die Mitverwendung von Inertmaterial. Außerdem verkompliziert diese Verfahrensweise in notwendiger Weise das Füllen der Kontaktrohre (eine Übersicht über die verschiedenen vorgeschlagenen Gegenmaßnahmen enthält beispielsweise die DE-PS 2 830 765). Eine weitere naheliegende Möglichkeit zur Minderung der Heißpunktbildung besteht darin, die Acrolein-Belastung des Reaktors zu reduzieren. Diese Maßnahme mindert jedoch gleichzeitig die Raum-Zeit-Ausbeute an gewünschtem Produkt.

Die DE-A 4 132 263 empfiehlt die katalytische Gasphasenoxidation von Acrolein zu Acrylsäure so durchzuführen, daß die Reaktionstemperatur in Strömungsrichtung längs der Kontaktrohre bis zum Erreichen eines Umsatzes des Acroleins von 20 bis 40 mol.-% 260 bis 300°C beträgt und anschließend die Reaktionstemperatur abrupt oder längs der Kontaktrohre bis zum Erreichen eines Acroleinumsatzes von ≥ 95 mol.-% sukzessive, stufenweise oder kontinuierlich um insgesamt 5 bis 40°C mit der Maßgabe gesenkt wird, daß die Reaktionstemperatur in dieser zweiten Reaktionszone nicht weniger als 240°C beträgt. Nachteilig an dieser Verfahrensweise ist jedoch, daß die Einstellung eines solchen Temperaturprofils die Anwendung von mehr als einem Wärmeaustauschmittelkreislauf erfordert.

Die DE-A 2 201 528 umfaßt für exotherme katalytische Vielkontaktrohr-Festbettoxidationen neben der Möglichkeit, das Wärmeaustauschmittel in einfacher Weise im wesentlichen direkt längs zu den Kontaktrohren zu führen auch die Möglichkeit, diese Längsführung lediglich über den gesamten Reaktionsbehälter betrachtet zu verwirklichen und dieser Längsströmung innerhalb des Reaktionsbehälters durch eine längs der Kontaktrohre aufeinderfolgende Anordnung von Durchtrittsquerschnitte frei lassenden Umlenkscheiben, eine Querströmung so zu überlagern, daß im Längsschnitt durch das Rohrbündel ein mäanderförmiger Strömungsverlauf des Wärmeaustauschmittels resultiert. Diesen Vorschlag umfassen auch die DE-PS 2 830 765, die DE-A 2 231 557 und die DE-A 2 310 517. Aus Trans I Chem. E, Vol. 71 Part B, August 1993, S. 208 bis 214 ist bekannt, daß bei exothermen katalytischen Vielkontaktrohr-Festbettoxidationen unübersichtliche indirekte Wechselwirkungen zwischen den Wärmeabgaben der individuellen Kontaktrohre erfolgen, weshalb die örtliche Lage des Heißpunktes sowie dessen Ausmaß in den individuellen Kontaktrohren in der Regel voneinander verschieden und vorausschauend kaum zu beurteilen ist.

Angesichts dieses Standes der Technik bestand die Aufgabe der vorliegenden Erfindung darin, ein neues Verfahren zur katalytischen Gasphasenoxidation von Acrolein zu Acrylsäure in einem Vielkontaktrohr-Festbettreaktor, durch dessen die Kontaktrohre umgebenden Raum lediglich ein Wärmeaustauschmittelkreislauf geleitet wird, bei erhöhter Temperatur an katalytisch aktiven Multimetalloxiden zur Verfügung zu stellen, das für ein gegebenes Acrolein enthaltendes Reaktionsgasgemisch bei gegebener Katalysatorbeschickung sowie vorgegebener Acroleinbelastung einen vorgegebenen Umsatz des Acroleins (bei einfachem Durchgang ≥ 95 mol.-% betragend) und eine vorgegebene Selektivität (≥ 90 mol.-%) der Acrylsäurebildung (d.h. eine vorgegebene Raum-Zeit-Ausbeute an Acrylsäure) in möglichst einfacher und günstiger Weise unter Ausbildung reduzierter Heißpunkttemperaturen zu liefern vermag.

Demgemäß wurde ein Verfahren zur katalytischen Gasphasenoxidation von Acrolein zu Acrylsäure in einem Vielkontaktrohr-Festbettreaktor, durch dessen die Kontaktrohre umgebenden Raum lediglich ein Wärmeaustauschmittelkreislauf geleitet wird, bei erhöhter Temperatur an katalytisch aktiven Multimetalloxiden mit einem Umsatz des Acroleins bei einfachem Durchgang von ≥ 95 mol.-% und einer Selektivität der Acrylsäurebildung von ≥ 90 mol.-% gefunden, das dadurch gekennzeichnet ist, daß man das Wärmeaustauschmittel einerseits über den Reaktionsbehälter betrachtet längs zu den Kontaktrohren im Gleichstrom zum Reaktionsgasgemisch durch den Vielkontaktrohr-Festbettreaktor leitet und andererseits innerhalb des Reaktionsbehälters durch eine längs der Kontaktrohre aufeinderfolgende Anordnung von Durchtrittsquerschnitte frei lassenden Umlenkscheiben eine Querströmung so tiberlagert, daß im Längsschnitt durch das Rohrbündel ein mäanderförmiger Strömungsverlauf des Wärmeaustauschmittels resultiert, mit der Maßgabe, daß die Strömungsgeschwindigkeit des im Kreis geführten Wärmeaustauschmittels so bemessen wird, daß die Temperatur des Wärmeaustauschmittels von der Eintrittsstelle in den Reaktor bis zur Austrittsstelle aus dem Reaktor um 2 bis 10°C, vorzugsweise um 3 bis 8°C und ganz besonders bevorzugt um 4 bis 6°C, ansteigt.

Die DE-PS 1 601 162 rät in Spalte 2 von einer solchen Ausführungsform ab, da mit ihr keine ausreichend gleichmäßigen Rohrtemperaturen über den Reaktorquerschnitt hinweg zu erreichen seien.

Erfindungsgemäß wird die Eintrittstemperatur des Wärmeaustauschmittels in den Reaktor in an sich bekannter Weise so gewählt, daß sich bei gegebener Katalysatorbeschickung und vorgegebener Acrolein-Belastung das zur Erzielung des geforderten Acrolein-Umsatzes sowie der geforderten Acrylsäure-Selektivität erforderliche Reaktionstemperaturprofil einstellt. Üblicherweise liegen die Reaktionstemperaturen eines solchen Profils bei Anwendung der für diesen Zweck bekanntermaßen eingesetzten Molybdän und Vanadium in oxidischer Form enthaltenden Multimetalloxidkatalysatoren bei 200 bis 350$^0$C. Entsprechend liegen bevorzugte Eintrittstemperaturen des Wärmeaustauschmittels bei 180°C bis 300°C. Solchermaßen geeignete Multimetalloxidkatalysatoren können beispielsweise den US-A 3 775 474, US-A 3 954 855, US-A 3 893 951 und US-A 4 339 355 entnommen werden. Ferner eignen sich in besonderer Weise die Multimetalloxidmassen der EP-A 427 508, DE-A 2 909 671, DE-C 3 151 805, der DE-AS 2 626 887 und der DE-A 4 302 991.

Eine Vielzahl der geeigneten Multimetalloxidkatalysatoren läßt sich unter der allgemeinen Formel I

$$Mo_{12}V_aW_bCu_cNi_dX^1_e X^2_f X^3_g X^4_h X^5_i O_n \quad (I),$$

in der die Variablen nachfolgende Bedeutung aufweisen:

| | |
|---|---|
| $X^1$ | ein oder mehrere Alkalimetalle, |
| $X^2$ | ein oder mehrere Erdalkalimetalle, |
| $X^3$ | Chrom, Mangan, Cer und/oder Niob, |
| $X^4$ | Antimon und/oder Wismut, |
| $X^5$ | Silicium, Aluminium, Titan und/oder Zirkonium, |
| a | 1 bis 6, |
| b | 0,2 bis 4, |
| c | 0,5 bis 6, |
| d | 0,2 bis 6, |
| e | 0 bis 2, |
| f | 0 bis 3 |
| g | 0 bis 5, |
| h | 0 bis 40, |
| i | 0 bis 40 und |
| n | eine Zahl, die durch die Wertigkeit und Häufigkeit der von Sauerstoff verschiedenen Elemente bestimmt wird, |

subsummieren.

Sie sind in an sich bekannter Weise erhältlich (siehe z.B. DE-A 4 302 991) und werden üblicherweise in Substanz zu Kugeln, Ringen oder Zylindern geformt oder auch in Gestalt von Schalenkatalysatoren, d.h. mit der Aktivmasse beschichteten vorgeformten inerten Trägerkörpern, eingesetzt. Selbstverständlich können sie aber auch in Pulverform als Katalysatoren angewendet werden.

Als Oxidationsmittel wird Sauerstoff verwendet. Wird als inertes Verdünnungsgas $N_2$ gewählt, erweist sich die Verwendung von Luft als Sauerstoffquelle besonders vorteilhaft.

In der Regel wird bei einem Acrolein:Sauerstoff:Wasserdampf:Inertgas-Volumenverhältnis (Nl) von 1:(1 bis 3):(0 bis 20):(3 bis 30), vorzugsweise von 1:(1 bis 3):(0,5 bis 10):(7 bis 18)gearbeitet. Normalerweise wird bei dem Verfahren Acrolein eingesetzt, das durch katalytische Gasphasenoxidation von Propen erzeugt wurde. In der Regel werden die Acrolein enthaltenden Reaktionsgase dieser Propenoxidation ohne Zwischenreinigung eingesetzt. Der Reaktionsdruck des erfindungsgemäßen Verfahrens beträgt in der Regel 1 bis 3 bar und die Gesamtraumbelastung beträgt vorzugsweise 1000 bis 2500 Nl/l/h.

Bei dem erfindungsgemäßen Verfahren wird keine reine Acrylsäure sondern ein Gasgemisch erhalten, von dessen Nebenkomponenten die Acrylsäure in an sich bekannter Weise abgetrennt werden kann.

In bezug auf Kontaktrohrmaterial, -dimensionierung, -anzahl, Rohrteilung und mögliche Wärmeaustauschmittel gilt für das erfindungsgemäße Verfahren das im Rahmen der Würdigung des Standes der Technik Gesagte. Als bevorzugtes Wärmeaustauschmittel wird erfindungsgemäß eine Salzschmelze bestehend aus 60 Gew.-% Kaliumnitrat (KNO$_3$) und 40 Gew.-% Natriumnitrit (NaNO$_2$) eingesetzt.

Zur Erzeugung der erfindungsgemäß erforderlichen Querströmung kann beispielsweise eine Anordnung von Umlenkscheiben angewendet werden, die abwechselnd an den gegenüberliegenden Seiten des Reaktorbehälters einen Durchtrittsquerschnitt frei lassen (vgl. z.B. DE-AS 1 039 040). Mit zunehmender Leistungsauslegung des Reaktors, bei der wegen der großen Zahl von Kontaktrohren auch das Verhältnis von Durchmesser zu Länge des Reaktionsbehälters entsprechend groß ist, wird jedoch eine Anordnung von Umlenkscheiben bevorzugt, die abwechselnd in der Mitte und an ihrem äußeren Rand (die Befestigung solcher Umlenkscheiben kann z.B. an einer im Reaktorzentrum vertikal angebrachten Stange erfolgen) einen Durchtrittsquerschnitt frei lassen (Zusatzmerkmal a), so daß das Wärmeaustauschmittel nacheinander jeweils von außen nach innen und von innen nach außen geleitet wird. Mit Vorteil wird man dabei im wesentlichen ringförmig angeordnete Rohrbündel (wobei im wesentlichen jedes Kontaktrohr mit Vorteil sechs äquidistante Nachbarn hat) mit einem freien zentralen Raum anwenden, wobei der Durchmesser des freien zentralen Raumes etwa 10 bis 30 % des Reaktorinnendurchmessers beträgt (Zusatzmerkmal b). Der Abstand der am weitesten außen liegenden Kontaktrohre zur Behälterwand wird normalerweise wenige cm betragen. Ferner werden die Kontaktrohre vorzugsweise an den Umlenkscheiben nicht abdichtend befestigt. Vielmehr werden mit Vorteil zwischen den Kontaktrohren und den Umlenkscheiben Spalte (Spaltbreite in der Regel <1 mm) so belassen, daß die Querströmungsgeschwindigkeit des Wärmeaustauschmittels innerhalb einer zwischen zwei aufeinanderfolgenden Umlenkscheiben befindlichen Zone möglichst konstant ist (Zusatzmerkmal c). In Verbindung mit unterschiedlichen Abständen der Umlenkscheiben läßt sich ferner mit Vorteil erreichen, daß die Temperaturdifferenzen (möglichst $\leq$ 3°C) und die Druckverluste in einem waagerechten Schnitt innerhalb einer Zone beschränkt werden (Zusatzmerkmal d). Des weiteren erweist es sich erfindungsgemäß als günstig, wenn der Ein- und Austritt des Wärmeaustauschmittels über an den beiden Enden des Behälters angebrachte Ringleitungen mit über den ganzen Umfang desselben verteilten Fenstern erfolgt, wobei die Fensteröffnungen so gestaltet sind, daß durch jedes Fenster pro Zeiteinheit die gleiche Menge an Wärmeaustauschmittel tritt (Zusatzmerkmal e), um eine möglichst gleichmäßige radiale Zu- bzw. Abführung des Wärmeaustauschmittels zu gewährleisten (vgl. DE-A 1 601 162).

Außerdem ist es erfindungsgemäß von Vorteil, wenn auf einer Umsatzhöhe von 20 bis 50 mol.-% umgesetztem Acrolein, vorzugsweise 30 bis 40 mol.-%, dem Reaktor eine Teilmenge des Wärmeaustauschmittels entnommen wird (z.B. über eine weitere Ringleitung zum Abführen), die vorzugsweise 30 bis 70 %, besonders bevorzugt 40 bis 60 %, der zugeführten Gesamtmenge an Wärmeaustauschmittel beträgt (Zusatzmerkmal f). Ferner wird das Reaktionsgasgemisch der Katalysatorbeschickung vorzugsweise auf die Eintrittstemperatur des Wärmeaustauschmittels vorerwärmt zugeführt (Zusatzmerkmal g). Dies läßt sich in einfacher Weise durch Durchströmung einer auf entsprechender Temperatur befindlichen Inertmaterialschüttung realisieren.

Bei erfindungsgemäß besonders vorteilhaften Verfahrensvarianten sind möglichst viele der Zusatzmerkmale a bis g simultan erfüllt. Besonders bevorzugt sind alle Zusatzmerkmale a bis g simultan erfüllt. Es wird diesseits davon ausgegangen, daß insbesondere bei der letztgenannten Verfahrensweise ein Temperaturverlauf in der Kontaktrohrwand längs eines einzelnen Kontaktrohres erzielt wird, bei dem die Temperatur der Kontaktrohrwand bis zu einem Acroleinumsatz von 20 bis 50 mol.-% im wesentlichen konstant ist und anschließend bis zum Rohrende um 2 bis 10°C anwächst. Es wird diesseits ferner davon ausgegangen, daß bei dieser Verfahrensweise im vorgenannten Umsatzbereich auch über den Reaktorquerschnitt im wesentlichen einheitliche Wandtemperaturen der Kontaktrohre vorliegen.

Ganz generell wird man versuchen die Anzahl an verwendeten Umlenkblechen zu beschränken. Anwendungstechnisch zweckmäßig beträgt diese Anzahl 3 bis 9.

Einen für die Durchführung der besonders vorteilhaften erfindungsgemäßen Verfahrensvariante geeigneten Reaktortyp zeigt die Fig. 1 der DE-AS 2 201 528.

Selbstverständlich kann die erfindundungsgemäße Verfahrensweise zur Minderung der Heißpunkttemperatur bei vorgegebener Raum-Zeit-Ausbeute mit den im Stand der Technik aufgeführten Verfahrensvorschlägen kombiniert angewendet werden.

Als besonders günstig erweist sich das erfindungsgemäße Verfahren, wenn das Beschickungsgasgemisch als inertes Verdünnungsgas ein solches enthält, das im wesentlichen, bevorzugt ausschließlich, aus brennbaren Gasen besteht, wie es in der in Deutschland unter dem Aktenzeichen 19508531.0 eingereichten Patentanmeldung beschrieben ist. Dies gilt insbesondere dann, wenn das Beschickungsgasgemisch gleichzeitig einen erhöhten Volumenanteil an $O_2$ und Propylen aufweist ("fette Fahrweise"). In diesem Zusammenhang bevorzugte inerte Verdünnungsgase sind Methan, Ethan, Propan, Butan, Pentan und deren Gemische (vgl. dazu die in Deutschland unter den Aktenzeichen 19508532.9 und 19508558.2 eingereichten Patentanmeldungen).

Umsatz U und Selektivität S sind in dieser Schrift wie folgt definiert

$$U \ (\text{mol.-\%}) = \frac{\text{Molzahl umgesetztes Acrolein}}{\text{Molzahl eingesetztes Acrolein}} \cdot 100$$

$$S \ (\text{mol.-\%}) = \frac{\text{Molzahl Acrolein umgesetzt zu Acrylsäure}}{\text{Molzahl Acrolein insgesamt umgesetzt}} \cdot 100$$

und auf einfachen Durchgang bezogen.

Beispiele

A) Verfahren zur katalytischen Gasphasenoxidation von Acrolein zu Acrylsäure in einem Vielkontaktrohr -Festbettreaktor bei denen das Wärmeaustauschmittel im wesentlichen direkt (unmittelbar) längs zu den Kontaktrohren geführt wird (Vergleichsbeispiele).

I. Beschreibung der allgemeinen Verfahrensbedingungen
Verwendetes Wärmeaustauschmittel: Salzschmelze, bestehend aus 50 Gew.-% Kaliumnitrat und 50 Gew.-% Natriumnitrit;
Material der Kontaktrohre: ferritischer Stahl;

Abmessungen der Kontaktrohre:
3200 mm Länge;
25 mm Innendurchmesser;
30 mm Außendurchmesser
(Wandstärke: 2,5 mm);
Anzahl der Kontaktrohre im Rohrbündel:
15700;
Reaktor:
Zylinderförmiger Behälter eines Innendurchmessers von 5000 mm;
Homogene Verteilung der Kontaktrohre auf dem gesamten Querschnitt mit einer Kontaktrohrteilung von 38 mm.
Die Kontaktrohre waren mit ihren Enden in Kontaktrohrböden der Dicke 100 mm abdichtend befestigt und mündeten mit ihren Öffnungen in je eine am oberen bzw. unteren Ende mit dem Behälter verbundene Haube.

Zuführung des Wärmeaustauschmittels zum Rohrbündel:
Über einen um den Reaktorbehälter (Reaktormantel) angebrachten Ringkanal. Über den Umfang des Reaktormantels angebrachte Fenster Einströmung in radialer Richtung zum Rohrbündel.
25 mm unterhalb des oberen Rohrbodens und 25 mm oberhalb des unteren Rohrbodens waren 10 mm dicke Trennbleche (Verteilerscheiben) angebracht, die sich über den gesamten Querschnitt erstreckten. Zwischen den Trennblechen und den Kontaktrohren bestanden durchlässige Spalte.
Die Salzschmelze trat zwischen dem unteren Rohrboden und dem unteren Trennblech in das Rohrbündel ein und verteilte sich über die Spalte über den Reaktorquerschnitt und stieg dann parallel zu den Kontakrohren nach oben. Die Salzschmelze strömte bei Erreichen des oberen Trennblechs durch die Spalte zwischen Trennblech und den Kontaktrohren hindurch und strömte dann in dem Raum zwischen dem oberen Trennblech und dem oberen Rohrboden radial zum äußeren Rohrkreis und sammelte sich über Fensterdurchtritte in einem oberen Ringkanal um den Reaktormantel und wurde nach Abkühlen auf die ursprüngliche Eintrittstemperatur durch eine Pumpe wiederum in den unteren Ringkanal gedrückt.
Die Wahl der Spaltbreiten erfolgte gemäß der DE-PS 1 601 162 u. DE-AS 1 675 501 so, daß sich für alle Stromfäden vom unteren zum oberen Ringkanal der gleiche hydraulische Widerstand ergab.
Kontaktrohrbeschickung: Schalenkatalysator gemäß Bsp. B1 der DE-A 43 02 991.

Struktur der Beschickung (von unten nach oben):
400 mm Schütthöhe nackte Katalysatorträger (Steatitkugeln des Durchmessers 5 mm),
800 mm Schalenkatalysator mit 17 Gew.-% Aktivmassenanteil,
2000 mm Schalenkatalysator mit 20 Gew.-% Aktivmassenanteil.
Belastung mit Reaktionsgasgemisch:
44750 Nm$^3$/h.
Zusammensetzung des Reaktionsgemisches:
4,2 Vol.-% Acrolein,
0,3 Vol.-% Acrylsäure,
5,5 Vol.-% Sauerstoff,
2,4 Vol.-% CO$_x$,
6,9 Vol.-% H$_2$O,
80,7 Vol.-% N$_2$.
Vorgegebene Umsetzungsdaten:

U = 99,5 mol.-%,
S = 95,5 mol.-%.
Raum-Zeit-Ausbeute:
180 kg Acrylsäure/m$^3$h.

II. Ergebnisse
Vorstehend gegebene Vorgaben konnten unter nachfolgenden Bedingungen realisiert werden:

| Bedingungen | Eintrittstempera-tur der Salz-schmelze | Austrittstempera-tur der Salz-schmelze | Heißpunkttempe-ratur | Pumpleistung (m$^3$/h) | Stromführung Salzschmelze relativ zum Reaktionsgasgemisch |
|---|---|---|---|---|---|
| a) | 257°C, Δ = 2°C | 259°C | 317°C | 3800 | Gegenstrom |
| b) | 263°C, Δ = 1°C | 264°C | 307°C | 7600 | Gleichstrom |
| c) | 259°C, Δ = 2°C | 261°C | 311°C | 3800 | Gleichstrom |
| d) | 248°C, Δ = 5°C | 253°C | 327°C | 1500 | Gleichstrom |

Die Heißpunkttemperatur wurde an 5 Kontaktrohren bestimmt, die im Rohrbündel äquidistant von ganz außen nach ganz innen radial liegend ausgewählt wurden. Die angegebene Temperatur gibt den höchsten ermittelten Heißpunktwert wieder.

Gegenstromführung von Salzschmelze und Reaktionsgasgemisch bedingt ganz offensichtlich ungünstige Heißpunkttemperaturen.

Bei Gleichstromführung werden die Heißpunktbedingungen mit zunehmender Pumpleistung, d.h. zunehmend verschwindender Temperaturdifferenz zwischen Eintritt- und Austrittstemperatur der Salzschmelze günstiger.

Unter den Bedingungen d) war kein stabiler kontinuierlicher Langzeitbetrieb des Reaktors mehr möglich.

B) Verfahren zur katalytischen Gasphasenoxidation von Acrolein zu Acrylsäure in einem Vielkontaktrohr-Festbettreaktor bei denen das Wärmeaustauschmittel im Längsschnitt durch das Kontaktrohrbündel mäanderförmig geführt wird.

I. Beschreibung der allgemeinen Verfahrensbedingungen
Verwendetes Wärmeaustauschmittel: wie AI;
Anzahl der Kontaktrohre im Rohrbündel: 25500;

Reaktor:    Zylinderförmiger Behälter eines Durchmessers von 6800 mm. Ringförmig angeordnetes Rohrbündel mit einem freien zentralen Raum. Durchmesser des zentralen freien Raumes: 1000 mm. Abstand der am weitesten außen liegenden Kontaktrohre zur Behälterwand: 150 mm. Homogene Kontaktrohrverteilung im Rohrbündel (6 äquidistante Nachbarrohre pro Kontaktrohr), Kontaktrohrteilung: 38 mm.

Die Kontaktrohre waren mit ihren Enden in Kontaktrohrböden der Dicke 125 mm abdichtend befestigt und mündeten mit ihre Öffnungen in je eine am oberen bzw. unteren Ende mit dem Behälter verbundenen Haube.

Zuführung des Wärmeaustauschmittels zum Rohrbündel:

Das Rohrbündel war durch drei zwischen den Kontaktrohrböden längs derselben aufeinanderfolgend angebrachte Umlenkscheiben (Dicke jeweils 10 mm) in vier äquidistante (jeweils 730 mm) Längsabschnitte (Zonen) geteilt.

Die unterste und die oberste Umlenkscheibe wies Ringgeometrie auf, wobei der Ringinnendurchmesser 1000 mm betrug und der Ringaußendurchmesser sich bis zur Behälterwand abdichtend erstreckte. Die Kontaktrohre waren an den Umlenkscheiben nicht abdichtend befestigt. Vielmehr waren eine Spaltbreite <0,5 mm aufweisende Spalte so belassen, daß die Querströmungsgeschwindigkeit der Salzschmelze innerhalb einer Zone möglichst konstant war.

Die mittlere Umlenkscheibe war kreisförmig und erstreckte sich bis zu den am weitesten außen liegenden Kontaktrohren des Rohrbündels.

Die Kreisführung der Salzschmelze wurde durch zwei Salzpumpen bewerkstelligt, von denen jede eine Rohrbündellängshälfte versorgte.

Die Pumpen drückten die Salzschmelze in einem um den Reaktormantel unten angebrachten Ringkanal, der die Salzschmelze über den Behälterumfang verteilte. Durch im Reaktormantel befindliche Fenster gelangte die Salzschmelze im untersten Längsabschnitt zum Rohrbündel. Die Salzschmelze floß dann der Vorgabe der Umlenkbleche folgend in der Abfolge

- von außen nach innen,
- von innen nach außen,
- von außen nach innen,
- von innen nach außen,

im wesentlichen mäanderförmig, über den Behälter betrachtet, von unten nach oben. Durch im obersten Längsabschnitt um den Behälterumfang angebrachte Fenster sammelte sich die Salzschmelze in einem oberen um den Reaktormantel angebrachten Ringkanal und wurde nach Abkühlung auf die ursprüngliche Eintrittstemperatur durch die Pumpen wieder in den unteren Ringkanal gedrückt.

Kontaktrohrbeschickung, Struktur der Beschickung, Zusammensetzung des Reaktionsgemisches und vorgegebene Umsetzungsdaten: wie AI.

Die Belastung mit Reaktionsgasgemisch: 72680 Nm$^3$/h.

II. Ergebnisse

Die vorgegebenen Umsetzungsdaten (Umsatz, Selektivität, Raum-Zeit-Ausbeute) konnten unter nachfolgenden Bedingungen realisiert werden:

| Bedingungen | Eintrittstemperatur der Salzschmelze | Austrittstemperatur der Salzschmelze | Heißpunkttemperatur | Pumpleistung (m$^3$/h) | Stromführung Salzschmelze relativ zum Reaktionsgasgemisch |
|---|---|---|---|---|---|
| a) | 267°C, Δ = 2°C | 269°C | 308°C | 6200 | Gegenstrom |
| b) | 266°C, Δ = 2°C | 268°C | 303°C | 6200 | Gleichstrom |
| c) | 265°C, Δ = 3°C | 268°C | 301°C | 4100 | Gleichstrom |
| d) | 263°C, Δ = 5°C | 268°C | 300°C | 2300 | Gleichstrom |
| e) | 262°C, Δ = 8°C | 270°C | 298°C | 1500 | Gleichstrom |
| f) | 260°C, Δ = 11°C | 271°C | 303°C | 1000 | Gleichstrom |
| g) | 258°C, Δ = 15°C | 273°C | 306°C | 800 | Gleichstrom |

Die Heißpunkttemperatur wurde an 5 Kontaktrohren bestimmt, die im Rohrbündel äquidistant von ganz außen nach ganz innen radial liegend ausgewählt wurden. Die angegebene Temperaturangabe gibt den höchsten ermittelten Heißpunktwert wieder.

Über den Reaktor betrachtete Gegenstromführung von Salzschmelze und Reaktionsgasgemisch bedingt hier in offensichtlicher Weise die ungünstigsten Heißpunkttemperaturen.

In überraschender Weise durchläuft das Heißpunktverhalten hier im Unterschied zu A) mit abnehmender Pumpleistung (Zunehmender Differenz zwischen Eintritts- und Austrittstemperatur des Wärmeaustauschmittels jedoch ein Minimum. Mit abnehmender Pumpleistung nehmen die Inhomogenitäten im Temperaturprofil des Reaktors (waagrechter Schritt) jedoch zu, weshalb aus Stabilitätsgründen ein Δ von 3 bis 8°C, vorzugsweise 4 bis 6°C, zwischen Ein- und Austrittstemperatur des Wärmeaustauschmittels bevorzugt wird.

Dieser überraschende Befund ist offensichtlich darauf zurückzuführen, daß der durch die Querströmungskomponente verbesserte Wärmeaustausch sowie der durch die reduzierte Eintrittstemperatur des Wärmeaustauschmittels erhöhte Kühleffekt im Bereich von unterhalb 50 mol.-% gelegenen Acroleinumsätzen das Heißpunktverhalten verbessert und die damit in diesem Abschnitt einhergehende Abnahme der Raum-Zeit-Ausbeute an Acrylsäure durch die durch die Wärmetönung der Reaktion bedingte Temperaturerhöhung im Bereich von Acroleinumsätzen oberhalb 50 mol.-% in überraschender Weise wieder ausgeglichen werden kann. Eine Grundlage für dieses Ergebnis dürfte sein, daß der Wärmeübergangskoeffizient auf der Wärmeträgerseite der Reaktionsrohre mit abnehmender Pumpleistung überraschend offensichtlich nicht im selben Ausmaß wie diese abnimmt.

Eine weitere Verbesserung wird daher dadurch möglich, daß man auf einer Umsatzhöhe von 20 bis 50 mol.-% umgesetztem Acrolein eine Teilmenge, vorzugsweise 30 bis 70 % der Zulaufmenge, des Wärmeaustauschmittels entnimmt. Dies bedingt eine noch bessere relative Kühlung sowie Vereinheitlichung der Temperatur über einen Reaktorquerschnitt im Bereich geringer Umsätze und gleichzeitig eine stärkere relative Temperaturerhöhung im Bereich hoher Umsätze.

Bei einer Zulauftemperatur der Salzschmelze von 264°C und einer Reduktion der umgepumpten Salzschmelze von 5400 $m^3$/h auf 2300 $m^3$/h (entnommene Teilmenge = 57 %) auf der Höhe der ersten (Acroleinumsatz = ca. 35 mol-%) Umlenkscheibe (Drosselventil) resultiert unter sonst gleichen Bedingungen, wie unter B aufgeführt, eine Heißpunkttemperatur von 297°C bei einer Austrittstemperatur von 269°C. Gleichzeitig verbessert eine solche Verfahrensweise die Homogenität des Temperaturprofils des Reaktors (waagrechter Schnitt) und die Homogenität der Lage der Heißpunkte in den individuellen Kontaktrohren. Eine abnehmende Pumpleistung ist gleichbedeutend mit einer erheblichen Kostenreduktion.

Im übrigen eröffnet das erfindungsgemäße Ergebnis die Option, entweder bei vorgegebener Raum-Zeit-Ausbeute aufgrund der günstigeren Heißpunktlage eine längere Standzeit der Katalysatorbeschickung zu erreichen, oder bei vorgegebener Standzeit durch Erhöhung der Belastung eine erhöhte Raum-Zeit-Ausbeute zu erzielen.

## Patentansprüche

1. Ein Verfahren zur katalytischen Gasphasenoxidation von Acrolein zu Acrylsäure in einem Vielkontaktrohr-Festbettreaktor, durch dessen die Kontaktrohre umgebenden Raum lediglich ein Wärmeaustauschmittelkreislauf geleitet wird, bei erhöhter Temperatur an katalytisch aktiven Multimetalloxiden mit einem Umsatz des Acroleins bei einfachem Durchgang von ≥ 95 mol.-% und einer Selektivität der Acrylsäurebildung von ≥ 90 mol.-%, dadurch gekennzeichnet, daß man das Wärmeaustauschmittel einerseits über den Reaktionsbehälter betrachtet längs zu den Kontaktrohren im Gleichstrom zum Reaktionsgasgemisch durch den Vielkontaktrohr-Festbettreaktor leitet und andererseits innerhalb des Reaktionsbehälters durch eine längs der Kontaktrohre aufeinderfolgende Anordnung von Durchtrittsquerschnitte frei lassenden Umlenkscheiben eine Querströmung so überlagert, daß im Längsschnitt durch das Kontaktrohrbündel ein mäanderförmiger Strömungsverlauf des Wärmeaustauschmittels resultiert, und dabei die Strömungsgeschwindigkeit des im Kreis geführten Wärmeaustauschmittels so bemißt, daß die Temperatur des Wärmeaustauschmittels von der Eintrittstelle in den Reaktor bis zur Austrittstelle aus dem Reaktor um 2 bis 10°C ansteigt.

2. Verfahren nach Anspruch 1, dadurch gekennzeichnet, daß die Temperatur des Wärmeaustauschmittels von der Eintrittstelle in den Reaktor bis zur Austrittstelle aus dem Reaktor um 3 bis 8°C ansteigt.

3. Verfahren nach Anspruch 1, dadurch gekennzeichnet, daß die Temperatur des Wärmeaustauschmittels von der Eintrittstelle in den Reaktor bis zur Austrittstelle aus dem Reaktor um 4 bis 6°C ansteigt.

4. Verfahren nach Anspruch 1 bis 3, dadurch gekennzeichnet, daß eine Anordnung von Umlenkscheiben verwendet wird, die abwechselnd in der Mitte und an ihrem äußeren Rand einen Durchtrittsquerschnitt frei lassen (Zusatzmerkmal a).

5. Verfahren nach Anspruch 1 bis 4, dadurch gekennzeichnet, daß man im wesentlichen ringförmig angeordnete Rohrbündel mit einem freien zentralen Raum anwendet.

6. Verfahren nach Anspruch 5, dadurch gekennzeichnet, daß der Durchmesser des freien zentralen Raumes etwa 10 bis 30 % des Reaktorinnendurchmessers beträgt (Zusatzmerkmal b).

7. Verfahren nach Anspruch 1 bis 6, dadurch gekennzeichnet, daß man die Kontaktrohre an den Umlenkscheiben nicht abdichtend befestigt, sondern zwischen Kontaktrohren und Umlenkscheiben Spalte beläßt.

8. Verfahren nach Anspruch 7, dadurch gekennzeichnet, daß man die Spaltbreiten so bemißt, daß die Querströmungsgeschwindigkeit des Wärmeaustauschmittels innerhalb einer zwischen zwei aufeinanderfolgenden Umlenkscheiben befindlichen Zone möglichst konstant ist (Zusatzmerkmal c).

9. Verfahren nach Anspruch 1 bis 8, dadurch gekennzeichnet, daß man durch nicht äquidistante Anordnung der Umlenkscheiben die Temperaturdifferenzen und die Druckverluste in einem waagrechten Schnitt innerhalb einer Zone beschränkt (Zusatzmerkmal d).

10. Verfahren nach Anspruch 1 bis 9, dadurch gekennzeichnet, daß der Ein- und Austritt des Wärmeaustauschmittels über an den beiden Enden des Reaktorbehälters angebrachte Ringleitungen mit über den ganzen Umfang desselben verteilten Fenstern erfolgt, wobei die Fensteröffnungen so gestaltet sind, daß durch jedes Fenster pro Zeiteinheit die gleiche Menge an Wärmeaustauschmittel tritt (Zusatzmerkmal e).

11. Verfahren nach Anspruch 1 bis 10, dadurch gekennzeichnet, daß auf einer Umsatzhöhe von 20 bis 50 mol.-% umgesetztem Acrolein dem Reaktor eine Teilmenge des Wärmeaustauschmittels entnommen wird.

12. Verfahren nach Anspruch 11, dadurch gekennzeichnet, daß die Entnahme auf einer Umsatzhöhe von 30 bis 40 mol.-% umgesetztem Acrolein erfolgt.

13. Verfahren nach Anspruch 11 oder 12, dadurch gekennzeichnet, daß die entnommene Teilmenge des Wärmeaustauschmittels 30 bis 70 % der zugeführten Gesamtmenge des Wärmeaustauschmittels beträgt (Zusatzmerkmal f).

14. Verfahren nach Anspruch 1 bis 13, dadurch gekennzeichnet, daß das Reaktionsgasgemisch der Katalysatorbeschickung auf die Eintrittstemperatur des Wärmeaustauschmittels vorerwärmt zugeführt wird (Zusatzmerkmal g).

15. Verfahren nach Anspruch 1 bis 14, dadurch gekennzeichnet, daß die Zusatzmerkmale a bis g simultan erfüllt sind.

16. Verfahren nach Anspruch 1 bis 15, dadurch gekennzeichnet, daß die Katalysatorbeschickung einen Molybdän und Vanadium in oxidischer Form enthaltenden Multimetalloxidkatalysator umfaßt.

17. Verfahren nach Anspruch 1 bis 16, dadurch gekennzeichnet, daß als Wärmeaustauschmittel eine Salzschmelze bestehend aus 60 Gew.-% Kaliumnitrat ($KNO_3$) und 40 Gew.-% Natriumnitrit ($NaNO_2$) eingesetzt wird.

## Claims

1. A process for the catalytic gas-phase oxidation of acrolein to acrylic acid in a multiple contact tube fixed-bed reactor through whose space surrounding the contact tubes only one heat-exchange medium circuit is passed, at elevated temperature on catalytically active multimetal oxides with an acrolein conversion for a single pass of $\geq$ 95 mol% and an acrylic acid formation selectivity of $\geq$ 90 mol%, which comprises firstly passing the heat-exchange medium through the multiple contact tube fixed-bed reactor longitudinally, considered over the reaction container as a whole, to the contact tubes in cocurrent to the reaction-gas mixture and secondly super-posing a transverse flow within the reaction container by means of an arrangement of successive baffles along the contact tubes which leaves passage cross sections free, so as to give a meandrous flow of the heat-exchange medium, seen in longitudinal section through the contact tube bundle, and setting the flow rate of the circulated heat-exchange medium so that its temperature rises by from 2 to 10°C between the point of entry into the reactor and the point of exit out of the reactor.

2. A process as claimed in claim 1, wherein the temperature of the heat-exchange medium rises by from 3 to 8°C between the point of entry into the reactor and the point of exit out of the reactor.

3. A process as claimed in claim 1, wherein the temperature of the heat-exchange medium rises by from 4 to 6°C between the point of entry into the reactor and the point of exit out of the reactor.

4. A process as claimed in claims 1 to 3, wherein an arrangement of baffles is used which leaves a passage cross section free alternately in the center and at its outer periphery (additional feature a).

5. A process as claimed in claims 1 to 4, wherein tube bundles having a free central space which are arranged in an essentially annular manner are used.

6. A process as claimed in claim 5, wherein the diameter of the free central space is from about 10 to 30% of the reactor internal diameter (additional feature b).

7. A process as claimed in claims 1 to 6, wherein the contact tubes are not attached to the baffles in a sealing manner,

10

but instead gaps are left between the contact tubes and the baffles.

8. A process as claimed in claim 7, wherein the gap widths are set so that the transverse flow rate of the heat-exchange medium within a zone located between two successive baffles is very constant (additional feature c).

9. A process as claimed in claims 1 to 8, wherein a non-equidistant arrangement of the baffles restricts the temperature differences and the pressure drops in a horizontal section within a zone (additional feature d).

10. A process as claimed in claims 1 to 9, wherein the entry and exit of the heat-exchange medium take place via ring pipelines which are attached to the two ends of the reactor container and have windows distributed over the entire periphery thereof, the window openings being designed in such a way that the same amount of heat-exchange medium passes through each window per time unit (additional feature e).

11. A process as claimed in claims 1 to 10, wherein a part-amount of heat-exchange medium is removed from the reactor at an acrolein conversion of from 20 to 50 mol%.

12. A process as claimed in claim 11, wherein the removal takes place at an acrolein conversion of from 30 to 40 mol%.

13. A process as claimed in claim 11 or 12, wherein the part-amount of heat-exchange medium removed is from 30% to 70% of the total amount of heat-exchange medium fed in (additional feature f).

14. A process as claimed in claims 1 to 13, wherein the reaction-gas mixture is fed to the catalyst charge after being pre-warmed to the entry temperature of the heat-exchange medium (additional feature g).

15. A process as claimed in claims 1 to 14, wherein additional features a to g are incorporated simultaneously.

16. A process as claimed in claims 1 to 15, wherein the catalyst charge comprises a multimetal oxide catalyst comprising molybdenum and vanadium in oxidic form.

17. A process as claimed in claims 1 to 16, wherein the heat-exchange medium is a salt melt consisting of 60% by weight of potassium nitrate ($KNO_3$) and 40% by weight of sodium nitrite ($NaNO_2$).

**Revendications**

1. Procédé d'oxydation catalytique en phase gazeuse d'acroléine en acide acrylique, à température élevée sur des oxydes métalliques complexes à activité catalytique, avec une conversion de l'acroléine $\geq$ 95 % en moles pour un passage et une sélectivité de la formation d'acide acrylique $\geq$ 90 % en moles, dans un réacteur à lit fixe et à tubes de contact multiples, une circulation d'agent d'échange de chaleur étant conduite à travers l'espace entourant les tubes de contact, caractérisé, d'une part, en ce que l'agent d'échange de chaleur vu du dessus du corps du réacteur, est conduit le long des tubes de contact, à cocourant avec le mélange gazeux réactionnel à travers le réacteur à lit fixe et à tubes de contact multiples et, d'autre part, en ce qu'on lui donne en plus une orientation transversale à l'intérieur du corps du réacteur, au moyen d'une succession de disques d'orientation percés d'orifices de passage et situés le long des tubes de contact, de sorte qu'il en résulte un parcours sinueux du courant d'agent d'échange de chaleur à travers le faisceau de tubes de contact, en coupe longitudinale, et ainsi on détermine la vitesse du courant d'agent d'échange de chaleur en circulation afin que la température de l'agent d'échange de chaleur s'élève de 2-10°C entre le point d'entrée dans le réacteur et le point de sortie du réacteur.

2. Procédé selon la revendication 1, caractérisé en ce que la température de l'agent d'échange de chaleur s'élève de 3-8°C entre le point d'entrée dans le réacteur et le point de sortie du réacteur.

3. Procédé selon la revendication 1, caractérisé en ce que la température de l'agent d'échange de chaleur s'élève de 4-6°C entre le point d'entrée dans le réacteur et le point de sortie du réacteur.

4. Procédé selon l'une quelconque des revendication 1 à 3, caractérisé en ce que l'on utilise une succession de disques d'orientation qui présentent un orifice de passage situé alternativement en leur milieu et à leur bord extérieur (caractéristique supplémentaire a).

5. Procédé selon l'une quelconque des revendications 1 à 4, caractérisé en ce que l'on utilise des faisceaux de tubes disposés principalement en forme d'anneau avec un espace central libre.

6. Procédé selon la revendication 5, caractérisé en ce que le diamètre de l'espace central libre s'élève à environ 10-30 % du diamètre interne du réacteur (caractéristique supplémentaire b).

7. Procédé selon l'une quelconque des revendications 1 à 6, caractérisé en ce que l'on ne fixe pas les tubes de contact aux disques d'orientation de façon jointive, mais en laissant un interstice entre les tubes de contact et les disques d'orientation.

8. Procédé selon la revendication 7, caractérisé en ce que l'on détermine les largeurs des interstices de sorte que la vitesse du courant transversal d'agent d'échange de chaleur soit le plus constante possible à l'intérieur d'une zone située entre deux disques d'orientation successifs (caractéristique supplémentaire c).

9. Procédé selon l'une quelconque des revendications 1 à 8, caractérisé en ce que l'on limite les différences de température et les pertes de charge, dans une coupe horizontale à l'intérieur d'une zone, par une disposition non-équidistante des disques d'orientation (caractéristique supplémentaire d).

10. Procédé selon l'une quelconque des revendications 1 à 9, caractérisé en ce que l'entrée et la sortie de l'agent d'échange de chaleur s'effectuent par des conduites annulaires placées aux deux extrémités du corps du réacteur, au moyen de fenêtres identiques réparties sur toute la circonférence, les ouvertures des fenêtres étant formées de toute la circonférence, les ouvertures des fenêtres étant formées de telle sorte que la même quantité d'agent d'échange de chaleur par unité de temps passe par chacune des fenêtres (caractéristique supplémentaire e).

11. Procédé selon l'une quelconque des revendications 1 à 10, caractérisé en ce que l'on retire une partie de l'agent d'échange de chaleur pour un niveau de conversion de l'acroléine mis dans le réacteur de 20-50 % en moles.

12. Procédé selon la revendication 11, caractérisé en ce que le retrait est effectué pour un niveau de conversion de l'acroléine de 30-40 % en moles.

13. Procédé selon la revendication 11 ou 12, caractérisé en ce que la partie de l'agent d'échange de chaleur retirée s'élève à 30-70 % de la quantité totale d'agent d'échange de chaleur introduit (caractéristique supplémentaire f).

14. Procédé selon l'une quelconque des revendications 1 à 13, caractérisé en ce que le mélange réactionnel gazeux est amené préchauffé à la température d'entrée de l'agent d'échange de chaleur vers la charge de catalyseur (caractéristique supplémentaire g).

15. Procédé selon l'une quelconque des revendications 1 à 14, caractérisé en ce que les caractéristiques supplémentaires a à g sont remplies simultanément.

16. Procédé selon l'une quelconque des revendications 1 à 15, caractérisé en ce que la charge de catalyseur comprend un catalyseur à oxyde métallique complexe contenant du molybdène et du vanadium, sous forme oxydée.

17. Procédé selon l'une quelconque des revendications 1 à 16, caractérisé en ce que l'on utilise en tant qu'agent d'échange de chaleur, un sel fondu constitué de 60 % en poids de nitrate de potassium ($KNO_3$) et de 40 % en poids de nitrite de sodium ($NaNO_2$).